# FASCICULE DE BREVET EUROPEEN

(11) **EP 1 620 116 B1**
(45) Date de publication et mention de la délivrance du brevet: **08.07.2009**
(21) Numéro de dépôt: 04742389.2
(22) Date de dépôt: 30.03.2004
(51) Int. Cl.: A61K 36/74, A61P 9/10

(54) **UTILISATION DU QUINQUINA POUR LA PREPARATION D'UN MEDICAMENT STIMULANT L'ANGIOGENESE**
VERWENDUNG DER CINCHONAPFLANZE ZUR HERSTELLUNG EINES MEDIKAMENTES ZUR STIMULIERUNG DER ANGIOGENESE
USE OF QUINQUINA FOR THE PREPARATION OF A MEDICAMENT STIMULATING ANGIOGENESIS

(30) Priorité: 01.04.2003 FR 0304003
(43) Date de publication de la demande: 01.02.2006
(73) Titulaire: Pierre Fabre Dermo-Cosmétique, 92100 Boulogne-Billancourt (FR)
(72) Inventeur: FABRE, Bernard, F-31450 Belberaud (FR); FORT-LACOSTE, Lydie, F-31860 Labarthe sur Leze (FR); JEANJEAN, Michel, F-31320 Castanet-Tolosan (FR)
(74) Mandataire: Ahner, Francis
(86) Numéro de dépôt international: PCT/FR2004/000788
(87) Numéro de publication internationale: WO 2004/089390

(56) Documents cités:
- EP-A- 0 394 920
- US-A- 4 568 546
- US-A- 5 006 338
- DATABASE WPI Section Ch, Week 199412 Derwent Publications Ltd., London, GB; Class D21, AN 1994-092754 XP002255454 & BR 9 203 054 A (DOS SANTOS M A) 25 janvier 1994 (1994-01-25)
- DATABASE WPI Section Ch, Week 200278 Derwent Publications Ltd., London, GB; Class B04, AN 2002-715505 XP002255455 & JP 2002 121106 A (NOEVIR KK) 23 avril 2002 (2002-04-23)
- DATABASE WPI Section Ch, Week 199642 Derwent Publications Ltd., London, GB; Class D21, AN 1996-421883 XP002255456 & JP 08 208451 A (KAO CORP) 13 août 1996 (1996-08-13)
- GILANI A ET AL: "Selective bradycardiac and dual antihypertensive activities of cinchonine: An alakalid from cinchona bark." JOURNAL OF HYPERTENSION, vol. 18, no. Suppl. 4, 2000, page S162, XP009017027 18th Scientific Meeting of the International Society of Hypertension;Chicago, Illinois, USA; August 20-24, 2000 ISSN: 0263-6352
- PRINZ A: "THE DISCOVERY OF THE CARDIAC EFFECTIVENESS OF CHINCHONA BARK AND ITS ALKALOIDS" WIENER KLINISCHE WOCHENSCHRIFT, vol. 102, no. 24, 1990, pages 721-723, XP009017067 ISSN: 0043-5325
- BARRETT B ET AL: "Medicinal plants, science, and health care" JOURNAL OF HERBS, SPICES AND MEDICINAL PLANTS 2001 UNITED STATES, vol. 8, no. 2-3, 2001, pages 1-36, XP009017028 ISSN: 1049-6475
- HELGA GÜTTLER, FRANKFURT: "Homoepathy in geriatrics. Specific features in the treatment of age-related complaints - Part 1" PZ PRISMA 2002 GERMANY, vol. 9, no. 1, 2002, pages 52-68, XP009017297 ISSN: 0945-5566
- DATABASE WPI Section Ch, Week 200055 Derwent Publications Ltd., London, GB; Class D13, AN 2000-581898 XP002255457 & JP 2000 201623 A (KAWAUCHI K) 25 juillet 2000 (2000-07-25)
- DATABASE WPI Section Ch, Week 200227 Derwent Publications Ltd., London, GB; Class D13, AN 2002-210803 XP002255458 & JP 2001 321122 A (KAWAUCHI K) 20 novembre 2001 (2001-11-20)

## Description

La présente invention concerne de manière générale les domaines cosmétique et pharmaceutique. Elle a plus particulièrement pour objet une nouvelle composition pharmaceutique ou cosmétique notamment capillaire comprenant dans un véhicule approprié inerte, un extrait végétal contenant du quinquina et qui stimule l'angiogénèse. La présente invention couvre plus précisément, l'utilisation d'un extrait de quinquina en tant que principe actif pour la préparation d'une composition pharmaceutique ou cosmétique destinée au traitement préventif et/ou curatif d'une affection nécessitant une stimulation de l'angiogénèse, telle que par exemple les désordres capillaires, le vieillissement cutané, l'ischémie organique ou tissulaire.

Depuis de nombreuses années, on s'est attaché à lutter contre les désordres touchant la croissance ou le maintien du cheveu et des structures dont il dépend. Par désordres capillaires, on entend toute modification touchant à la structure ou au fonctionnement du follicule pileux. Plus particulièrement, par désordres capillaires, on entend toute perturbation biologique entraînant la perte occasionnelle ou prolongée des cheveux. Les désordres capillaires peuvent être d'origine occasionnelle ou installée.

Les désordres capillaires peuvent avoir différentes causes d'origines internes ou externes. Par origines internes, on entend toute perturbation touchant les processus biologiques impliqués dans la croissance et le maintien du cheveu. On citera par exemple le métabolisme hormonal, le dérèglement sébacé, l'amplification enzymatique des 5-α-réductases, l'inflammation, les modifications structurales du collagène, l'intervention des facteurs de croissance, des facteurs nerveux ou des facteurs vasculaires et la composante génétique. Par origines externes, on entend, l'incidence de la flore locale du cuir chevelu, l'action d'agents chimiques (colorations, permanentes, chimiothérapie), la pollution ou l'hygiène de vie.

Tous ces désordres, d'origines internes ou externes, ciblant le cuir chevelu, ont pour conséquence directe la perturbation et la modification du cycle pilaire conduisant à différentes formes d'alopécies. Le terme d'alopécie résume l'ensemble des pathologies atteignant le follicule pileux et le cycle de croissance de la tige pilaire et ayant pour conséquence la perte occasionnelle et réversible ou la chute définitive de tout ou partie des cheveux. On citera les 2 types d'alopécies les plus répandus, l'alopécie aérata et l'alopécie androgénétique ou androgénique.

Depuis longtemps, les scientifiques recherchent à travers différentes voies biologiques, les solutions visant à stimuler la croissance du cheveu ou à prolonger son attachement au cuir chevelu. Cette préoccupation est d'autant plus marquée dans le domaine de l'alopécie androgénique et plus particulièrement fréquente chez les sujets masculins, chez lesquels on constate une perturbation du cycle de croissance pilaire, entraînant la chute du cheveu.

Le follicule pileux constitue l'unité biologique fondamentale, directement impliquée dans le développement et le maintien du système pilaire. Il rassemble différentes entités structurales telles que la papille dermique, la glande sébacée, les gaines épithéliales, l'innervation ou la vascularisation impliquées séparément ou conjointement dans le cycle de développement des cheveux. Le lien existant entre la papille dermique et le bulbe reste déterminant dans la croissance du cheveu (cf. Olivier RF. Histological studies of whisker regeneration in the hooded rat. J. Embryol. Exp. Morphol. 1966 ; 311 :560-562).

Le cycle de croissance pilaire est divisé en trois phases principales :
- Une phase anagène active ou phase de croissance pendant laquelle le follicule s'enfonce dans les tissus dermiques. Les cellules du bulbe entament une mitose rapide suivie d'une différenciation. La phase anagène est reconnue aujourd'hui comme l'étape primordiale de la croissance du cheveu, son accélération expliquant le raccourcissement de la vie du cheveu et sa rapidité de chute.
- Une phase catagène au cours de laquelle les divisions cellulaires régressent puis s'arrêtent. A ce stade, le follicule pileux amorce une remontée extra-dermique et une rétraction, c'est la phase la plus courte estimée à environ 3 semaines.
- Une phase de repos ou phase télogène pendant laquelle le cheveu est progressivement délogé par un nouveau cheveu anagène issu du même follicule. Le cheveu natif va provoquer la chute du cheveu mature.

Il existe une perte de cheveux normale et physiologique de l'ordre de 60 à 100 par jour pour une chevelure saine. Au-delà, la chute est dite pathologique qu'elle soit occasionnelle ou installée.

A ce jour, on a proposé différents produits pour lutter contre les deux types d'alopécies. La plupart associent plusieurs principes actifs susceptibles d'apporter une action bénéfique sur les paramètres biologiques impliqués dans la chute des cheveux. Parmi les principes actifs les plus couramment rencontrés, nous pouvons citer à titre d'exemples :
- des vitamines telles que les vitamines A, E,B5, B6, C, H, PP,
- des oligo-éléments tels que le zinc, le cuivre, le magnésium, le silicium, etc.,
- des dérivés protéiques tels que les peptides, les acides aminés soufrés (type méthionine, cystine, cystéïne ou dérivés),
- des huiles essentielles ou des extraits d'origine végétale de nature lipophile ou hydrophile dont la liste n'est pas limitative, des agents antifongiques tels que la piroctonolamine, les dérivés undécyléniques, la cyclopiroxolamine, ...
- des molécules de synthèse chimique réputées pour leur action spécifique au niveau des récepteurs androgéniques ou sur la synthèse ou l'expression des 5-α-réductases.

Parmi les découvertes effectuées ces dernières années, le MINOXIDIL® (ou 2,4 diamino-6-pipéridinopyrimidine 3-oxide) fait aujourd'hui référence dans le traitement de l'alopécie androgénique. En dépit des nombreuses théories évoquées sur son mécanisme d'action, ce dernier n'est pas clairement élucidé. De plus, son efficacité reste limitée même s'il est constaté une stabilisation de la chute dans de nombreux cas cliniques en raison de la reprise du processus alopéciant dès l'arrêt du traitement. Son usage journalier contraignant est vraisemblablement à l'origine d'effets secondaires indésirables relevés chez des patients l'utilisant à long terme tels que réactions cutanées localisées ou effets systémiques.

Dernièrement, on a proposé dans les demandes de brevet W096/09048 et W092/01437 une nouvelle molécule inspirée de la précédente la 2,4 diaminopyrimidine 3-oxide ou AMINEXIL®. L'AMINEXIL agit contre la fibrose périfolliculaire en inhibant l'expression de la lysyl-hydroxylase, elle-même responsable de la transformation accélérée de l'hydroxylysine conduisant à la formation précoce de collagène mature. Cette surproduction de collagène et simultanément sa réticulation entraînent une régidification de la gaine conjonctive et une fibrose périfolliculaire s'opposant aux processus de formation des néo-cheveux.

Si la croissance du poil dépend de plusieurs facteurs, les facteurs susceptibles d'induire une angiogénèse sont des candidats de choix dans l'induction et le maintien de la vascularisation au niveau de la papille dermique du follicule pileux qui est la structure contrôlant la croissance pilaire se caractérisant par la présence d'un réseau vasculaire très développé en phase anagène (cf. Richard A. Ellis, Guiseppe Moretti. Vascular patterns associated with catagen hair follicules in the human scalp. Annals New York Academy of Sciences, 1959-83, 448-457). Il a été démontré que les cellules de cette papille expriment durant ce stade et de façon intense, un facteur de croissance angiogénique le VEGF (vascular endothélial growth factor) (S. Lachgar -M. Charveron in « Hair for the next millenium » - Ed S.D, JJ Van Neste - VA. Randall - H. Baden - H. Ogawa et R. Oliver - Elsevier - Amsterdam - 1996 - p. 407-412). Cette découverte est d'autant plus importante qu'il y a disparition du réseau capillaire durant le passage du stade anagène au stade catagène.

Ainsi, le développement pilaire semble être lié au maintien des capillaires sanguins puisque la chute du poil est associée à une disparition du réseau capillaire de la papille durant la phase télogène. Ainsi, le brevet EP 1 082 125 protège l'utilisation d'un extrait de Ruscus aculeatus qui stimule la production du VEGF dans des compositions pharmaceutiques ou cosmétiques capillaires.

Le problème à résoudre à la base de l'invention est le maintien et/ou la stimulation de la croissance des cheveux exposés à une chute anormale comme c'est le cas dans les alopécies aérata ou androgéniques. Plus précisément, le problème à la base de l'invention est d'identifier de nouveaux actifs susceptibles de stimuler l'angiogénèse et ainsi d'être utilisés dans des compositions pharmaceutiques ou cosmétiques, notamment capillaires. Les inventeurs ont maintenant mis en évidence de manière fortuite l'action d'un extrait d'écorce de quinquina rouge sur la stimulation de l'angiogénèse.

Compte-tenu des implications multiples de la présente invention, il convient de préciser que l'invention ne se limite pas aux applications capillaires, pharmaceutiques ou cosmétiques. Elle s'étend également aux multiples applications cosmétiques tel que le traitement topique externe des affections relevant des désordres de l'angiogénèse, notamment soins du visage ou soins corporels et par exemple du traitement du vieillissement cutané (cf. Stefan Frank et al. The Journal of Biological Chemistry, vol. 270, n° 21, pp. 12607-12613, 1996). Elle s'étend également aux multiples applications pharmaceutiques nécessitant une stimulation de l'angiogénèse, telles que par exemple la cicatrisation des tissus, le traitement de l'ischémie tissulaire, notamment cardiaque.

La présente invention a dont donc pour objet l'utilisation d'un extrait de quinquina en tant que principe actif pour la préparation d'une composition pharmaceutique ou cosmétique destinée au traitement préventif et/ou curatif d'une affection nécessitant une stimulation de l'angiogénèse, **caractérisée en ce que** la dite affection est sélectionnée parmi :
- les désordres capillaires,
- le vieillissement cutané,

Par extrait de quinquina, on entend désigner le produit d'extraction d'une ou de plusieurs sortes de quinquinas de préférence obtenus à partir des écorces de quinquinas (pour revue sur les quinquinas, voir Dorvault, l'Officine, 23ème Edition Vigot pp1471-1478).

L'extrait de quinquina de la présente invention est choisi parmi la quarantaine d'espèces de quinquina connues à ce jour, entre lesquelles les hybridations sont nombreuses. De préférence, le quinquina selon l'invention est choisi parmi :
- les quinquinas gris (Chinchona officinalis) tels les types Loxa et Huanuco, qui sont aromatiques, pauvres en tanin et en alcaloïdes,
- les quinquinas jaunes (Chinchona calisaya) qui sont les quinquinas les plus riches en alcaloïdes totaux et en quinine,
- les quinquinas rouges (Chinchona succirubra) qui sont riches en tanin, et plus riches que les quinquinas gris en alacaloïdes,
- les quinquinas à quinine.

Des extraits d'écorce de quinquina sont connus pour certaines propriétés thérapeutiques. Ainsi, les écorces de quinquinas sont astringentes par leur tanin, toniques amères, fébrifuges et anti-malariques par leurs alcaloïdes, notamment par la quinine. Elle possède une action dépressive sur les centres de la thermogenèse des sujets fébriles, d'où son emploi comme antipyrétique. Les propriétés antipyrétiques de l'écorce sont dues principalement à la quinine, et dans une moindre mesure aux alcaloïdes quinidine, cinchonine, cinchonidine. Le quinquina est tonique grâce à son acide quinotannique qui est combiné en partie avec des alcaloïdes. L'action tonique est aussi due à la quinovine. En dehors de ses propriétés antimalariques, elle possède une action anti-infectieuses et anti-grippale. Outre l'activité anti-infectieuse, antiseptique qui explique l'intérêt des poudres ou extraits de quinquina dans le soin des plaies ou des ulcères, le quinquina a été peu utilisé par voie topique.

Les quinquinas gris et jaunes sont essentiellement utilisés en thérapeutique pour leurs propriétés fébrifuges, sous la forme de tisane à 2% par infusion de 1 heure. Le quinquina rouge est inscrit sur la liste des tisanes et est surtout utilisé pour ses propriétés toniques (poudre de Lucas-Championnière et poudre dentifrice).

De manière préférée, le quinquina selon l'invention est choisi parmi les quinquinas rouges (Chinchona succirubra).

La composition selon l'invention contient un extrait de quinquina utilisé à titre de principe actif et non pas de tonique comme il a pu parfois être utilisé dans certaines compositions cosmétiques (EP 1 082 125). De fait, sa concentration dans la dite composition est avantageusement d'au moins 1 % en poids d'extrait de quinquina, de préférence d'environ 1,5%, d'environ 2,5%, d'environ 3%, d'environ 5%, d'environ 7,5%, d'environ 10%, d'environ 12,5%, d'environ 15% en poids.

Les compositions pharmaceutiques peuvent être formulées de manières conventionnelles bien connues de l'homme de l'art en utilisant un ou plusieurs véhicules pharmaceutiquement ou cosmétiquement acceptables comprenant des excipients, adjuvants et des auxiliaires tels par exemple des agents conservateurs, des stabilisants, des agents de mouillage ou des émulsifiants etc. La méthode de formulation choisie dépend de la voie d'administration désirée. La dite composition comprend en outre un véhicule pharmaceutiquement ou cosmétiquement acceptable.

Les procédés de préparation d'extraits de quinquina sont bien connus de l'homme du métier (voir Dorvault, l'Officine, 238"' Edition Vigot pp1471-1478). A titre d'illustration, des exemples non limitatifs de procédés d'extraction sont donnés ci-après.

La composition chimique d'extraits de quinquina est fort complexe et peut varier d'une espèce à l'autre. Néanmoins, les extraits de quinquina contiennent les quatre alcaloïdes suivants : la cinchonine et son isomère la cinchonidine, la quinine et son isomère la quinidine. A ces alcaloïdes, il convient d'ajouter d'autres composés présents en concentration diverses selon l'espèce de quinquina : la cupréine, l'hydrocinchonine, l'hydrocinchonidine, la cinchonamine, l'hydroquinine, l'hydroquinidine, la quinamine, la paricine, l'aricine, la cinchovaline, la cusconine, la paltochine, la carthagine, la pseudo-quinine, la paytine, la cinchogénine, l'acide quinique, l'acide cinchotannique ou quinotannique, et le quinovose (isomère du rhamnose).

L'invention concerne l'utilisation d'extraits de quinquina dont le degré d'extraction et de purification peut varier. Il entre également dans l'étendue de l'invention de purifier les extraits de quinquina afin de les enrichir en composé(s) présentant une action stimulante de l'angiogenèse.

Par angiogénèse, on entend désigner la croissance et le développement de nouveaux vaisseaux sanguins. Le processus de l'angiogénèse est un processus complexe impliquant la rupture des membranes vasculaires basales, la migration et la prolifération des cellules endothéliales, puis subséquemment la formation de vaisseaux sanguins et leur maturation.

Selon un premier mode de réalisation, la présente invention a des applications dans les domaines cosmétiques, dermo-cosmétiques et/ou dermatologiques. Plus particulièrement, l'invention concerne l'utilisation d'un extrait de quinquina pour la fabrication d'une composition cosmétique, dermo-cosmétique et/ou dermatologique topique destinée au traitement des affections relevant des désordres angiogéniques. L'invention porte également sur le procédé de traitement cosmétique, dermocosmétique et/ou dermatologique des affections relevant de désordres angiogéniques, caractérisé en ce que l'on applique une composition comprenant un véhicule approprié et une dose efficace d'un extrait végétal contenant du quinquina et qui stimule l'angiogénèse.

Ainsi, l'invention concerne l'utilisation de la composition pharmaceutique et/ou cosmétique pour le traitement préventif et/ou curatif des désordres capillaires. De préférence, les désordres capillaires sont sélectionnés parmi l'alopécie aérata et l'alopécie androgénogénétique ou androgénique. En outre, la composition selon l'invention contient des excipients pharmacologiquement et/ou cosmétologiquement acceptables et sélectionnés de telle sorte à améliorer sinon préserver l'esthétique capillaire du patient. Plus particulièrement, l'invention a pour objet l'utilisation d'un extrait végétal dans un véhicule adapté en vue de son application sur le cuir chevelu de façon réitérée, de sorte à provoquer une stimulation du bulbe folliculaire en phase anagène donc une croissance du cheveu.

Selon ce premier mode de réalisation, la présente invention concerne également l'utilisation de la composition pharmaceutique et/ou cosmétique pour le traitement topique externe du vieillissement cutané. En outre, la composition contient des excipients pharmacologiquement et/ou cosmétologiquement acceptables destinés à un usage topique externe.

Dans ce premier mode de réalisation, la composition se présente sous la forme de lotions, gels, mousses, shampoings, émulsions huile dans eau, émulsions eau dans huile, éventuellement bi- ou tri-phasiques, crèmes, pommades. Des exemples non limitatifs de ces compositions sont donnés ci-après.

Selon un second mode de réalisation, la présente invention a des applications thérapeutiques préventives ou curatives. En effet, il reste un besoin important de développer de nouveaux composés et méthodes capables d'induire et de stimuler l'angiogénèse dans un organe ou un tissu cible. En effet, l'afflux de sang dans un organe ou un tissu peut être atténué suite à une maladie, un traumatisme, une intervention chirurgicale, lors de la cicatrisation tissulaire, ou lors d'autres évènements. Le traitement préventif et/ou curatif d'un tel défaut d'approvisionnement en sang d'un organe ou d'un tissu est donc également l'objet de la présente invention.

Il entre également dans la portée de la présente invention d'utiliser l'extrait de quinquina selon l'invention ou au moins un des ses composés actifs comme produits de combinaison avec un second composé actif, de préférence stimulant l'angiogénèse, pour une utilisation simultanée, séparée ou échelonnée dans le temps pour le traitement préventif et/ou curatif des affections précédemment évoquées. Parmi lesdits seconds composés actifs il convient de citer de manière non exhaustive les facteurs de croissance connus pour stimuler l'angiogénèse tels le Fibroblast Growth Factor (FGF), le Transforming Growth Factor (TGF), le Placental Growth factor, le Platelet-derived Growth factor et le Vascular Endothelial Growth Factor (VEGF), mais également des cytokines tels l'interleukine IL8 et le Tumor Necrosis Factor (TNF), ainsi que toutes molécules pouvant stimuler ces substances.

Les compositions pharmaceutiques peuvent être formulées de manières conventionnelles bien connues de l'homme de l'art en utilisant un ou plusieurs supports physiologiquement acceptables comprenant des excipients, adjuvants et des auxiliaires tels par exemple des agents conservateurs, des stabilisants, des agents de mouillage ou d'émulsifiant. La méthode de formulation choisie dépend de la voie d'administration désirée. La quantité d'extrait de quinquina, ou de composition à administrer au patient dépend de l'affection à traiter, de l'effet visé, notamment un effet thérapeutique ou prophylactique, de l'état de santé et de l'âge du patient et du mode d'administration du médicament. Les quantités efficaces thérapeutiques ou prophylactiques à administrer à un patient humain peuvent être déterminées à partir de modèles animaux ou de données connues de l'homme du métier.

Les exemples qui suivent sont destinés à illustrer certains modes d'utilisation particuliers de l'invention. Les excipients mentionnés dans les formulations ne sont nullement limitatifs et il est facile pour l'homme de l'art d'en substituer d'autres.

### EXEMPLES

### Exemple 1 : la plante

Les quinquinas sont originaires de l'Amérique du Sud, où ils croissent à l'état sauvage dans la Cordillère des Andes. Au XVIIe siècle, des missionnaires espagnols, après la découverte du Pérou, ont reconnu les propriétés fébrifuges des écorces de Quinquinas, en relation avec leur amertume (à l'époque, tout ce qui était amer était réputé fébrifuge). En 1640 la comtesse de Chinchon (d'où Cinchona) aurait été guérie des fièvres par ces écorces et serait ainsi à l'origine de la réputation de la drogue et de son introduction en Europe.

Le quinquina fut d'abord introduit en Espagne, puis en Angleterre et en France, mais comme remède secret, sous les noms de "poudre de la Comtesse", "poudre des Jésuites" (parce que ces derniers furent, en 1649, les premiers qui en firent le commerce), "poudre du Cardinal" (le Cardinal de Lugo l'ayant introduit à Rome et ayant préconisé son usage).

Un anglais, du nom de Talbor (nom francisé en Talbot), ayant découvert le secret des Jésuites, se mit à leur faire concurrence, d'abord dans son pays, puis en France. Il put guérir des fièvres des grands personnages comme Charles II d'Angleterre, le Grand Dauphin de France, la reine d'Espagne. Enfin Louis XIV lui acheta son secret en 1679, et le fit publier.

L'usage s'en répandit, mais ce ne fut qu'en 1737 que grâce à La Condamine, on eut connaissance de l'arbre qui fournit cette précieuse écorce. C'est d'après les dessins et les descriptions de La Condamine que Linné, en 1742, créa le gendre Cinchona. Le mot quinquina vient de kina-kina, mots péruviens qui signifient écorces des écorces.

Les écorces de quinquina sont fournies par diverses espèces du genre Cinchona, de la famille des Rubiacées. Les cinchonas qui comptent une quarantaine d'espèces, sont des arbustes ou des petits arbres toujours verts pouvant atteindre 15 à 20 m, à feuilles opposées, pétiolées, nervurées, en forme de coeur allongé, pourvues à la base de 2 petites stipules souvent caduques...

Les fleurs, régulières, blanches, roses, pourpres, très odorantes, sont disposées en groupe à l'extrémité de petits rameaux. Elles sont souvent d'odeur agréable. Les parties employées sont, les écorces, riches en alcaloïdes divers (quinine, cinchonine, cupréïne, etc.). On distingue plusieurs variétés : les quinquinas gris, les quinquinas jaunes et les quinquinas rouges, cette dernière étant aujourd'hui la plus utilisée.

Les écorces de quinquinas renferment 8 à 10 % d'eau, 4 à 5 % de matières minérales, un peu d'amidon et de matières gommeuses, des traces d'huiles essentielles, des stérols (β-sitostérol). L'acide quinique (hexahydrotétrahydroxybenzoïque) a été séparé de ces écorces dès 1790, sa constitution n'ayant été établie qu'environ un siècle plus tard. Les tannins catéchiques (acide quinotannique) représentent 3 à 5 % des écorces. Leur oxydation donne un phlobaphène, "le rouge de quinquina".

Un principe amer, la quinovine (glucoside) a été isolé en 1821. Ce quinovoside est hydrolysable par les acides en un sucre particulier le quinovose et acide quinovique. Cette substance intervient avec les alcaloïdes dans l'amertume de la drogue. La quinovine brute renferme, à côté du gulométhyloside de l'acide quinovique, celui d'un acide voisin, l'acide cincholique, ainsi que le glucoside de l'acide quinovique.

Les alcaloïdes sont abondants, ils représentent 3 à 15 % des écorces de quinquinas cultivés. Les quatre alcaloïdes principaux sont : la cinchonine, la quinine, la quinidine et cinchonidine. Tous sont des dérivés de la quinoléine, il s'agit de deux couples de stéréo-isomères (quinine et quinidine d'une part et cinchonine et cinchonidine d'autre part). Les alcaloïdes s'accumulent dans les écorces surtout celles de la base du tronc. Au cours du développement de la plante, la teneur en alcaloïdes augmente rapidement, le maximum étant atteint vers 7 ans. La teneur en alcaloïdes totaux des principales espèces utilisées varie de 3 à 15 %.

### Exemple 2 : l'extraction du quinquina

### 2.1. Protocole général

Les écorces de quinquina sont préalablement séchées puis broyées afin de permettre l'extraction. Cette dernière est réalisée à l'aide d'un solvant comme l'eau, un alcool de C₁ à C₄, d'une cétone ou d'un mélange eau-alcool C₁ à C₄ ou eau-cétone en toutes proportions Le rapport plante/solvant varie de 1/3 à 1/20. L'extraction peut être menée sous agitation ou statiquement. La température d'extraction varie de la température d'ébullition du solvant à la température ambiante et sa durée s'étale de 1 heure à 24 heures.

Une fois l'extraction réalisée, les solutions sont récupérées par une séparation solide/liquide adéquate : filtration, décantation, centrifugation, essorage, etc.

Une deuxième extraction peut être menée sur le marc. Les paramètres entrent alors dans les plages décrites ci-dessus pour la première extraction. Les deux solutions sont alors jointes. Elles peuvent être utilisées telles quelles ou subir un traitement complémentaire. Pour ce faire, les solutions sont évaporées sous pression réduite à des températures situées entre 40°C et 100°C. Cette concentration peut être menée jusqu'à évaporation complète du solvant et obtention d'une poudre après séchage. Une concentration partielle des solutions peut permettre l'utilisation d'un concentrat. Ce dernier ainsi que la poudre obtenue après séchage peut être utilisé tel quel ou solubilisé par un solvant cosmétiquement compatible comme le propylène glycol, le butylène glycol.

Les extraits sont dosés pour leur teneur en quinine par CLHP. Les valeurs se situent entre 1 et 20 % pour 100 g de matière sèche de l'extrait.

### 2.2. Exemples d'extraction

a) Une tonne d'écorce sèche de quinquina est broyée puis extraite par 7 tonnes d'alcool absolu pendant 1 heure à reflux et sous agitation. La solution est récupérée par filtration, le marc est lavé par 1 tonne d'alcool absolu. L'extrait est dosé à 10 g en quinine pour 100 g de matière sèche de l'extrait liquide.
b) 100 kg d'écorce sèche de quinquina sont broyés puis extraits par 1 tonne d'isopropanol/eau (50/50) à température ambiante sous agitation pendant 4 heures. La solution extraite est récupérée par filtration. Le marc est re-extrait dans les mêmes conditions. Les solutions extraites sont jointes puis concentrées jusqu'à l'obtention d'un concentrat. Ce dernier est concentré à sec dans une étuve. On obtient ainsi 15 kg d'une poudre titrant 3 % en quinine.
c) 1 kg d'écorce sèche de quinquina est extrait par 10 kg de méthanol à 60° C pendant 1 heure. La solution organique récupérée par filtration est concentrée. On y ajoute 1 kg de propylène glycol puis on concentre de façon à retirer tout le méthanol. La solution de propylène glycol obtenu est filtrée puis dosée en quinine, sa teneur est de 8 g pour 100 g de matière sèche.

### Exemple 3 : effet de l'extrait de quinquina sur la néo-vascularisation

L'influence de l'extrait de quinquina préparé selon l'exemple 2 sur la néo-vascularisation a été évaluée sur un modèle d'angiogénèse in vitro.

Il s'agit d'une co-culture de cellules endothéliales humaines et de fibroblastes humains (kit angiogénèse Bioprédic International), qui est réalisée en présence ou pas des actifs à tester. Les cellules endothéliales migrent à travers la matrice pour former des structures tubulaires, qui sont quantifiées au bout de 9 jours de culture. La quantification de la longueur des capillaires est réalisée par analyse d'image.

| | Longueur des tubules par champ de comptage | |
|---|---|---|
| | Moyenne | Pourcentage d'augmentation / témoin |
| Témoin non traité | 6152 | - |
| Réf. positive (VEGF 10ng/ml) | 9090 | + 48 |
| Réf. Négative (Suramine 20-tM) | 3807 | - 38% |
| Extrait de quinquina 1 µg/ml | 10416 | + 69% |
| Extrait de quinquina 10 µg/ml | 12119 | + 97% |

Ainsi, l'extrait de quinquina favorise in vitro la néo-angiogénèse de manière dose-dépendante. '

### Exemple 4 : Formulations topiques à usage capillaire

### 4.1. LOTION BIPHASIQUE ANTICHUTE

| | | |
|---|---|---|
| PHASE LIPOPHILE A | | (35 %) |
| EXTRAIT HUILEUX DE SERENOA REPENS | | QS |
| ALPHA TOCOPHERYL NICOTINATE | | 1,5 % |
| LINOLEATE D'ETHYLE | | QS |
| ALPHA-PINENE | | QS |
| ETHANOL | | QS |
| DECAMETHYLCYCLOPENTASILOXANE | QSP | 100 g |
| PHASE HYDROPHILE B | | (65 %) |
| NICOTINAMIDE | | 0,5 % |
| LACTAMIDE MEA | | 0,75 % |
| EXTRAIT DE QUINQUINA | | 5 % |
| VITAMINE B6 (CHLORHYDRATE) | | 0,5 % |
| GLUCONATE DE ZINC | | 0,15 % |
| PIROCTONOLAMINE | | 0,075% |
| ALCOOL ISOPROPYLIQUE | | QS |
| EAU DEMINERALISEE STERILE | QSP | 100 g |

### 4.2. TRAITEMENT POUR LA CHUTE DES CHEVEUX

Application simultanée de 2 solutions

| **SOLUTION 1** | | |
|---|---|---|
| EXTRAIT DE QUINQUINA | | 3,5 % |
| ALPHA TOCOPHERYL NICOTINATE | | 0,1 % |
| PIROCTONOLAMINE | | 0,05 % |
| NICOTINAMIDE | | 1 % |
| ESSENCE EUCALYPTUS | | 0,05 % |
| HEXYLENE GLYCOL | | QS |
| ETHANOL | | QS |
| DECAMETHYLCYCLOPENTASILOXANE | QSP | 100 g |

| **SOLUTION 2** | | |
|---|---|---|
| ALPHA PINENE | | 3 % |
| ACIDE LAURIQUE | | 0,25 % |
| ACIDE BETA GLYCYRRHETINIQUE | | 0,30 % |
| BIOTINE | | 0,005 % |
| HEXYLENE GLYCOL | | QS |
| PROPYLENE GLYCOL | | QS |
| ETHANOL | QSP | 100 g |

### 4.3. SÉRUM ANTICHUTE - VIEILLISSEMENT

| | | |
|---|---|---|
| EXTRAIT DE QUINQUINA | | 5 % |
| D-PANTHENOL | | 0,4 % |
| PYRIDOXINE CHLORHYDRATE | | 0,10 % |
| ZINC GLUCONATE | | 0,25 % |
| PHYTOFLAVONES | | 1 % |
| ALPHA PINENE | | 2,5 % |
| ALPHA TOCOPHERYL NICOTINATE | | 0,15 % |
| PEG 40 HYDROGENATED CASTOR OIL | | 0,60 % |
| ALCOOL | QS | 35% vol |
| EAU DEMINERALISEE STÉRILE | QSP | 100 g |

### 4.4 : SOIN CREME APRES SHAMPOOING ANTICHUTE

| | | |
|---|---|---|
| EXTRAIT DE QUINQUINA | | 7 % |
| HYDROLYSAT DE PROTÉINE DE SOIE (sol. 20%) | | 2 % |
| D-PANTHENOL | | 1 % |
| BEHENYLTRIMMONIUM CHLORURE | | QS |
| CYCLOMETHICONE | | QS |
| BEURRE DE KARITE | | QS |
| HYDROXYPROPYLTRIMMONIUM GUAR | | QS |
| HUILE ESSENTIELLE DE ROMARIN | | QS |
| ALPHA TOCOPHERYL NICOTINATE | | 0,3 % |
| ACIDE CITRIQUE PH 4.5 | | QS |
| EAU DEMINERALISEE STÉRILE | QSP | 100 g |

### 4.5 : SHAMPOOING CREME RELAIS ANTICHUTE

| | | |
|---|---|---|
| ALPHA TOCOPHERYL NICOTINATE | | 0,5 % |
| EXTRAIT DE QUINQUINA | | 3,5 % |
| D PANTHENOL | | 0,3 % |
| SODIUM LAURETH SULFATE (28%) | | 25 % |
| SODIUM COCOAMPHOACETATE (30%) | | 12 % |
| COCAMIDOPROPYL BETAINE (30%) | | 7 % |
| PEG 7 GLYCERYL COCOATE | | QS |
| PEG 150 DISTEARATE | | QS |
| GLYCERYL PALMITATE | | 0,75 % |
| ETHYLENE GLYCOL DISTEARATE | | 0,75 % |
| HYDROXY PROPYL GUAR | | 0,075 % |
| ACIDE CITRIQUE pH 6,5 | | |
| CONSERVATEURS | | QS |
| EAU DEMINERALISEE STERILE | QSP | 100 g |

### 4.6 : LOTION CAPILLAIRE USAGE FREQUENT

| | | |
|---|---|---|
| ALPHA TOCOPHERYL NICOTINATE | | 0,2 % |
| DIGUANOSINE TETRAPHOSPHATE SOLUTION | | 0,75 % |
| ACETAMIDE MEA | | 0,75 % |
| SULFATE DE ZINC | | 0,5 % |
| D PANTHENOL | | 0,15 % |
| PYRIDOXINE CHLORHYDRATE | | 0,20 % |
| EXTRAIT DE QUINQUINA | | 1,5 % |
| DIMETHICONE COPOLYOL | | QS |
| ALCOOL 96% VOL. | | 35% vol |
| EAU DEMINERALISEE | QSP | 100 g |

### 4.7 : TRAITEMENT ANTICHUTE BIPHASIQUE

| | | |
|---|---|---|
| ACIDE BETA GLYCYRRHETINIQUE | | 0,3 % |
| HEXYLENE GLYCOL | | 10 % |
| ETHANOL 96° | | 33 % |
| DIETHYLPHTALATE | | 0,50 % |
| CHLORURE DE SODIUM | | 0,1 % |
| DIMETHICONE COPOLYOL | | 0,1 % |
| EXTRAIT FLUIDE DE PFAFFIA | | 4 % |
| DIGUANOSINE TETRAPHOSPHATE LYOPHILISAT | | 0,05 % |
| EAU DEMINERALISEE STERILE | | 5 % |
| EXTRAIT FLUIDE DE QUINQUINA | | 2,5 % |
| CROTAMITON | | 0,15 % |
| CLYCLOMETICONE | QSP | 100 g |

### 4.8 : GEL RUBEFIANT POUR TRAITER LA PELADE

| | | |
|---|---|---|
| ALPHA TOCOPHERYL NICOTINATE | | 2,2 % |
| EXTRAIT DE QUINQUINA | | 10 % |
| HUILE ESSENTIELLE DE CEDRE | | 0,2 % |
| HUILE ESSENTIELLE DE NIAOULI | | 0,15 % |
| PEG-40 HYDROGENATED CASTOR OIL | | 1,75 % |
| ETHOXYDIGLYCOL | | 7 % |
| CARBOMER | | QS |
| AMINOMETHYL PROPANOL | | QS |
| EAU DEMINERALISEE | | 15 % |
| ETHANOL | QSP | 100 g |

Quelques modèles types ont été décrits. Il est évident que la liste n'est pas limitative et qu'elle concerne toute forme galénique à usage capillaire pour le soin du cheveu et du cuir chevelu, mais plus généralement toute forme galénique à usage topique externe pour le traitement des affections relevant des désordres de l'angiogénèse tels par exemple le vieillissement cutané.

## Revendications

1. Utilisation d'un extrait de quinquina en tant que principe actif pour la préparation d'une composition pharmaceutique destinée au traitement préventif et/ou curatif des désordres capillaires par stimulation de l'angiogénèse.

2. Utilisation selon la revendication 1, **caractérisée en ce que** ledit quinquina est un quinquina rouge (Cinchona succirubra).

3. Utilisation selon l'une des revendications 1 à 2, **caractérisée en ce que** ladite composition contient au moins 1 % en poids d'extrait de quinquina et un véhicule pharmaceutiquement acceptable.

4. Utilisation selon l'une des revendications 1 à 3, **caractérisée en ce que** les désordres capillaires sont sélectionnés parmi l'alopécie aérata et l'alopécie androgénogénétique ou androgénique.

5. Utilisation d'un extrait de quinquina dans une composition cosmétique pour le traitement topique externe du vieillissement cutané.

6. Utilisation selon l'une des revendications 1 à 5, **caractérisée en ce que** ladite composition se présente sous la forme de lotions, gels, mousses, shampoings, émulsions huile-dans-eau, émulsions eau-dans-huile, éventuellement bi- ou tri-phasiques, crèmes, pommades.

## Claims

1. Use of a quinquina extract as an active ingredient for preparing a pharmaceutical composition intended for preventive and/or curative treatment of hair disorders by stimulation of angiogenesis.

2. The use according to claim 1, **characterized in that** said quinquina is a red quinquina (*Cinchona succirubra*).

3. The use according to any of claims 1 to 2, **characterized in that** said composition contains at least 1% by weight of quinquina extract and a pharmaceutically acceptable carrier.

4. The use according to any of claims 1 to 3, **characterized in that** the hair disorders are selected from alopecia areata and androgenetic or androgenic alopecia.

5. Use of a quinquina extract in a cosmetic composition for external topical treatment of skin ageing.

6. The use according to any of claims 1 to 5, **characterized in that** said composition appears as lotions, gels, foams, shampoos, oil-in-water emulsions, water-in-oil emulsions, optionally bi- or triphasic emulsions, creams, pomades.

## Patentansprüche

1. Verwendung eines Cinchonaextrakts als Wirkstoff für die Zubereitung einer pharmazeutischen Zusammensetzung zur vorbeugenden und/oder Heilbehandlung von Störungen des Haars durch Stimulierung der Angiogenese.

2. Verwendung nach Anspruch 1, **dadurch gekennzeichnet, dass** der besagte Cinchona ein roter Cinchona (Cinchona succirubra) ist.

3. Verwendung nach einem der Ansprüche 1 bis 2, **dadurch gekennzeichnet, dass** die Zusammensetzung mindestens 1 Gew.-% Cinchonaextrakt und einen pharmazeutisch akzeptablen Arzneiträger enthält.

4. Verwendung nach einem der Ansprüche 1 bis 3, **dadurch gekennzeichnet, dass** die Störungen des Haars aus dem kreisrunden Haarausfall und dem androgenetischen oder androgenen Haarausfall ausgewählt sind.

5. Verwendung eines Cinchonaextrakts in einer kosmetischen Zusammensetzung zur externen topischen Behandlung der Hautalterung.

6. Verwendung nach einem der Ansprüche 1 bis 5, **dadurch gekennzeichnet, dass** sich die Zusammensetzung in Form von Lotionen, Gelen, Schäumen, Shampoons, Ölin-Wasser-Emulsionen, Wasser-in-Öl-Emulsionen, eventuell zwei- oder dreiphasig, Cremes, Pomaden präsentiert.
